# EUROPÄISCHE PATENTSCHRIFT

(11) **EP 1 161 535 B1**
(45) Veröffentlichungstag und Bekanntmachung des Hinweises auf die Patenterteilung: **21.05.2008**
(21) Anmeldenummer: 00918695.8
(22) Anmeldetag: 08.03.2000
(51) Int. Cl.: C12N 15/12, C07K 14/47, C12N 9/00, C07K 16/18, C12Q 1/68, G01N 33/68, C12N 15/11, A61K 31/70, A61K 38/17

(54) **RNA POLYMERASE I TRANSKRIPTIONSFAKTOR TIF-IA**
RNA POLYMERASE I TRANSCRIPTION FACTOR TIF-IA
FACTEUR DE TRANSCRIPTION TIF-IA DE L'ARN POLYMERASE I

(30) Priorität: 17.03.1999 DE 19911992
(43) Veröffentlichungstag der Anmeldung: 12.12.2001
(73) Patentinhaber: Deutsches Krebsforschungszentrum Stiftung des öffentlichen Rechts, 69120 Heidelberg (DE)
(72) Erfinder: GRUMMT, Ingrid, D-69118 Heidelberg (DE); VINGRON, Martin, D-69123 Heidelberg (DE)
(74) Vertreter: Schüssler, Andrea
(86) Internationale Anmeldenummer: PCT/DE2000/000767
(87) Internationale Veröffentlichungsnummer: WO 2000/055316

(56) Entgegenhaltungen:
- YAMAMOTO ROBERT T ET AL: "RRN3 gene of Saccharomyces cerevisiae encodes an essential RNA polymerase I transcription factor which interacts with the polymerase independently of DNA template." EMBO (EUROPEAN MOLECULAR BIOLOGY ORGANIZATION) JOURNAL, Bd. 15, Nr. 15, 1996, Seiten 3964-3973, XP002143816 ISSN: 0261-4189
- SCHNAPP ANDREAS ET AL: "Function of the growth-regulated transcription initiation factor TIF-IA in initiation complex formation at the murine ribosomal gene promoter." MOLECULAR AND CELLULAR BIOLOGY, Bd. 13, Nr. 11, November 1993 (1993-11), Seiten 6723-6732, XP000929632 ISSN: 0270-7306 in der Anmeldung erwähnt
- D. BUTTGEREIT ET AL: "Growth-dependent regulation of rRNA synthesis is mediated by a transcription initiation factor (TIF-IA)" NUCLEIC ACIDS RESEARCH., Bd. 13, Nr. 22, 25. November 1985 (1985-11-25), Seiten 8165-8180, XP002143818 OXFORD UNIVERSITY PRESS, SURREY., GB ISSN: 0305-1048 in der Anmeldung erwähnt
- NCI-CGAP: "National cancer institute, cancer genome anatomy project (CGAP), " EMBL DATABASE ENTRY HSAA25257, ACCESSION NUMBER AA213789, 3. Februar 1997 (1997-02-03), XP002143819
- MOOREFIELD BETH ET AL: "RNA polymerase I transcription factor Rrn3 is functionally conserved between yeast and human." PROCEEDINGS OF THE NATIONAL ACADEMY OF SCIENCES OF THE UNITED STATES, Bd. 97, Nr. 9, 25. April 2000 (2000-04-25), Seiten 4724-4729, XP002143820 April 25, 2000 ISSN: 0027-8424

### SEQUENZPROTOKOLL

(1) ALLGEMEINE ANGABEN:
   (i) ANMELDER:
      (A) NAME: Deutsches Krebsforschungszentrum
      (B) STRASSE: Im Neuenheimer Feld 280
      (C) ORT: Heidelberg
      (E) LAND: Deutschland
      (F) POSTLEITZAHL: 69120
   (ii) BEZEICHNUNG DER ERFINDUNG: RNA Polymerase I Transkriptionsfektor TIF-IA
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER JETZIGEN ANMELDUNG:
      ANMELDENUMMER: DE 199 11 992.9
(2) ANGABEN ZU SEQ ID NO: 1:
   (i) SEQUENZKENNZEICHEN:
      (A) LÄNGE: 2040 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (ix) MERKMAL:
      (A) NAME/SCHLÜSSEL: CDS
      (B) LAGE:40..1992
   (xi) SEQUENZBESCHREIBUNG: SEQ ID NO: 1:
   GGAGCGGCCG CCCAGGTGCG GTCGCGTTAGT TCGGCCCA ATG GCG GCA CCG CTG         54
                                              Met Ala Ala Pro Leu
                                               1               5
   CTT CAC ACG CGT TTG CCG GGA GAT GCG GCC GCT TCG TCC TCT GCA GTT       102
   Leu His Thr Arg Leu Pro Gly Asp Ala Ala Ala Ser Ser Ser Ala Val
                   10                  15                  20
   AAG AAG CTG GGC GCG TCG AGG ACT GGG ATT TCA AAT ATG CGT GCA TTA       150
   Lys Lys Leu Gly Ala Ser Arg Thr Gly Ile Ser Asn Met Arg Ala Leu
               25                  30                  35
   GAG AAT GAC TTT TTC AAT TCT CCC CCA AGA AAA ACT GTT CGG TTT GGT       198
   Glu Asn Asp Phe Phe Asn Ser Pro Pro Arg Lys Thr Val Arg Phe Gly
           40                  45                  50
   GGA ACT GTG ACA GAA GTC TTG CTG AAG TAC AAA AAG GGT GAA ACA AAT       246
   Gly Thr Val Thr Glu Val Leu Leu Lys Tyr Lys Lys Gly Glu Thr Asn
       55                  60                  65
   GAC TTT GAG TTG TTG AAG AAC CAG CTG TTA GAT CCA GAC ATA AAG GAT       294
   Asp Phe Glu Leu Leu Lys Asn Gln Leu Leu Asp Pro Asp Ile Lys Asp
    70                  75                  80                  85
   GAC CAG ATC ATC AAC TGG CTG CTA GAA TTC CGT TCT TCT ATC ATG TAC        342
   Asp Gln Ile Ile Asn Trp Leu Leu Glu Phe Arg Ser Ser Ile Met Tyr
                   90                  95                  100
   TTG ACA AAA GAC TTT GAG CAA CTT ATC AGT ATT ATA TTA AGA TTG CCT        390
   Leu Thr Lys Asp Phe Glu Gln Leu Ile Ser Ile Ile Leu Arg Leu Pro
               105                 110                 115
   TGG TTG AAT AGA AGT CAA ACA GTA GTG GAA GAG TAT TTG GCT TTT CTT        438
   Trp Leu Asn Arg Ser Gln Thr Val Val Glu Glu Tyr Leu Ala Phe Leu
           120                 125                 130
   GGT AAT CTT GTA TCA GCA CAG ACT GTT TTC CTC AGA CCG TGT CTC AGC        486
   Gly Asn Leu Val Ser Ala Gln Thr Val Phe Leu Arg Pro Cys Leu Ser
       135                 140                 145
   ATG ATT GCT TCC CAT TTT GTG CCT CCC CGA GTG ATC ATT AAG GAA GGC        534
   Met Ile Ala Ser His Phe Val Pro Pro Arg Val Ile Ile Lys Glu Gly
   150                 155                 160                 165
   GAT GTA GAT GTT TCA GAT TCT GAT GAT GAA GAT GAT AAT CTT CCT GCA        582
   Asp Val Asp Val Ser Asp Ser Asp Asp Glu Asp Asp Asn Leu Pro Ala
                   170                 175                 180
   AAT TTT GAC ACA TGT CAC AGA GCC TTG CAA ATA ATA GCA AGA TAT GTA        630
   Asn Phe Asp Thr Cys His Arg Ala Leu Gln Ile Ile Ala Arg Tyr Val
               185                 190                 195
   CCA TCG ACA CCG TGG TTT CTC ATG CCA ATA CTG GTG GAA AAA TTT CCA        678
   Pro Ser Thr Pro Trp Phe Leu Met Pro Ile Leu Val Glu Lys Phe Pro
           200                 205                 210
   TTT GTT CGA AAA TCA GAG AGA ACA CTG GAA TGT TAC GTT CAT NAC TTA        726
   Phe Val Arg Lys Ser Glu Arg Thr Leu Glu Cys Tyr Val His Xaa Leu
       215                 220                 225
   CTA AGG ATT AGT GTA TAT TTT CCA ACC TTG AGG CAT GAA ATT CTG GAG        774
   Leu Arg Ile Ser Val Tyr Phe Pro Thr Leu Arg His Glu Ile Leu Glu
   230                 235                 240                 245
   CTT ATT ATT GAA AAA CTA CTC AAG TTG GAT GTG AAT GCA TCC CGG CAG        822
   Leu Ile Ile Glu Lys Leu Leu Lys Leu Asp Val Asn Ala Ser Arg Gln
                   250                 255                 260
   GGT ATT GAA GAT GCT GAA GAA ACA GCA ACT CAA ACT TGT GGT GGG ACA        870
   Gly Ile Glu Asp Ala Glu Glu Thr Ala Thr Gln Thr Cys Gly Gly Thr
               265                 270                 275
   GAT TCC ACG GAA GGA TTG TTT AAT ATG GAT GAA GAT GAA GAA ACT GAA        918
   Asp Ser Thr Glu Gly Leu Phe Asn Met Asp Glu Asp Glu Glu Thr Glu
           280                 285                 290
   CAT GAA ACA AAG GCT GGT CCT GAA CGG CTC GAC CAG ATG GTG CAT CCT        966
   His Glu Thr Lys Ala Gly Pro Glu Arg Leu Asp Gln Met Val His Pro
       295                 300                 305
   GTA GCC GAG CGC CTG GAC ATC CTG ATG TCT TTG GTT TTG TCC TAC ATG       1014
   Val Ala Glu Arg Leu Asp Ile Leu Met Ser Leu Val Leu Ser Tyr Met
   310                 315                 320                 325
   AAG GAT GTC TGC TAT GTA GAT GGT AAG GTT GAT AAC GGC AAA ACA AAG       1062
   Lys Asp Val Cys Tyr Val Asp Gly Lys Val Asp Asn Gly Lys Thr Lys
                   330                 335                 340
   GAT CTA TAT CGC GAC CTG ATA AAC ATC TTT GAC AAA CTC CTG TTG CCC       1110
   Asp Leu Tyr Arg Asp Leu Ile Asn Ile Phe Asp Lys Leu Leu Leu Pro
               345                 350                 355
   ACC CAT GCC TCC TGC CAT GTA CAG TTT TTC ATG TTT TAC CTC TGT AGT      1158
   Thr His Ala Ser Cys His Val Gln Phe Phe Met Phe Tyr Leu Cys Ser
           360                 365                 370
   TTC AAA TTG GGA TTC GCA GAG GCA TTT TTG GAA CAT CTC TGG AAA AAA      1206
   Phe Lys Leu Gly Phe Ala Glu Ala Phe Leu Glu His Leu Trp Lys Lys
       375                 380                 385
   TTG CAG GAC CCA AGT AAT CCT GCC ATC ATC AGG CAG GCT GCT GGA AAT      1254
   Leu Gln Asp Pro Ser Asn Pro Ala Ile Ile Arg Gln Ala Ala Gly Asn
   390                 395                 400                 405
   TAT ATT GGA AGC TTT TTG GCA AGA GCT AAA TTT ATT CCT CTT ATT ACT      1302
   Tyr Ile Gly Ser Phe Leu Ala Arg Ala Lys Phe Ile Pro Leu Ile Thr
                   410                 415                 420
   GTA AAA TCA TGC CTA GAT CTT TTG GTT AAC TGG CTG CAC ATA TAC CTT      1350
   Val Lys Ser Cys Leu Asp Leu Leu Val Asn Trp Leu His Ile Tyr Leu
               425                 430                 435
   AAT AAC CAG GAT TCG GGA ACA AAG GCA TTC TGC GAT GTT GCT CTC CAT      1398
   Asn Asn Gln Asp Ser Gly Thr Lys Ala Phe Cys Asp Val Ala Leu His
           440                 445                 450
   GGA CCA TTT TAC TCA GCC TGC CAA GCT GTG TTC TAC NCC TTT GTT TTT      1446
   Gly Pro Phe Tyr Ser Ala Cys Gln Ala Val Phe Tyr Xaa Phe Val Phe
       455                 460                 465
   AGA CAC AAG CAG CTT TTG AGC GGA AAC CTG AAA GAA GGT TTG CAG TAT      1494
   Arg His Lys Gln Leu Leu Ser Gly Asn Leu Lys Glu Gly Leu Gln Tyr
   470                 475                 480                 485
   CTT CAG AGT CTG AAT TTT GAG CGG ATA GTG ATG AGC CAG CTA AAT CCC      1542
   Leu Gln Ser Leu Asn Phe Glu Arg Ile Val Met Ser Gln Leu Asn Pro
                   490                 495                 500
   CTG AAG ATT TGC CTG CCC TCA GTG GTT AAC TTT TTT GCT GCA ATC ACA      1590
   Leu Lys Ile Cys Leu Pro Ser Val Val Asn Phe Phe Ala Ala Ile Thr
               505                 510                 515
   AAT AAG TAC CAG CTC GTC TTC TGC TAC ACC ATC ATT GAG AGG AAC AAT      1638
   Asn Lys Tyr Gln Leu Val Phe Cys Tyr Thr Ile Ile Glu Arg Asn Asn
           520                 525                 530
   CGC CAG ATG CTG CCA GTC ATT AGG AGT ACC GCT GGA GGA GAC TCA GTG      1686
   Arg Gln Met Leu Pro Val Ile Arg Ser Thr Ala Gly Gly Asp Ser Val
       535                 540                 545
   CAG ATC TGC ACA AAC CCG CTG GAC ACC TTC TTC CCC TTT GAT CCC TGT      1734
   Gln Ile Cys Thr Asn Pro Leu Asp Thr Phe Phe Pro Phe Asp Pro Cys
   550                 555                 560                 565
   GTG CTG AAG AGG TCA AAG AAA TTC ATT GAT CCT ATT TAT CAG GTG TGG      1782
   Val Leu Lys Arg Ser Lys Lys Phe Ile Asp Pro Ile Tyr Gln Val Trp
                   570                 575                 580
   GAA GAC ATG AGT GCT GAA GAG CTA CAG GAG TTC AAG AAA CCC ATG AAA      1830
   Glu Asp Met Ser Ala Glu Glu Leu Gln Glu Phe Lys Lys Pro Met Lys
               585                 590                 595
   AAG GAC ATA GTG GAA GAT GAA GAT GAT GAC TTT CTG AAA GGC GAA GTG      1878
   Lys Asp Ile Val Glu Asp Glu Asp Asp Asp Phe Leu Lys Gly Glu Val
           600                 605                 610
   CCC CAG AAT GAT ACC GTG ATT GGG ATC ACA CCA AGC TCC TTT GAC ACG      1926
   Pro Gln Asn Asp Thr Val Ile Gly Ile Thr Pro Ser Ser Phe Asp Thr
       615                 620                 625
   CAT TTC CGA AGT CCT TCA AGT AGT GTG GGC TCC CCA CCC GTG TTG TAC       1974
   His Phe Arg Ser Pro Ser Ser Ser Val Gly Ser Pro Pro Val Leu Tyr
   630                 635                 640                 645
   ATG CAA CCC AGT CCC CTC TGACGGCAGA AATTTGTGACTG AGATGTGACA           2024
   Met Gln Pro Ser Pro Leu
                   650
   TTTGGGATTC CCCATC                                                    2040
(2) ANGABEN ZU SEQ ID NO: 2:
   (i) SEQUENZKENNZEICHEN:
      (A) LÄNGE: 651 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ala Ala Pro Leu Leu His Thr Arg Leu Pro Gly Asp Ala Ala Ala
    1               5                  10                  15
   Ser Ser Ser Ala Val Lys Lys Leu Gly Ala Ser Arg Thr Gly Ile Ser
               20                  25                  30
   Asn Met Arg Ala Leu Glu Asn Asp Phe Phe Asn Ser Pro Pro Arg Lys
           35                  40                  45
   Thr Val Arg Phe Gly Gly Thr Val Thr Glu Val Leu Leu Lys Tyr Lys
       50                  55                  60
   Lys Gly Glu Thr Asn Asp Phe Glu Leu Leu Lys Asn Gln Leu Leu Asp
   65                  70                  75                      80
   Pro Asp Ile Lys Asp Asp Gln Ile Ile Asn Trp Leu Leu Glu Phe Arg
                   85                  90                  95
   Ser Ser Ile Met Tyr Leu Thr Lys Asp Phe Glu Gln Leu Ile Ser Ile
               100                 105                 110
   Ile Leu Arg Leu Pro Trp Leu Asn Arg Ser Gln Thr Val Val Glu Glu
           115                 120                 125
   Tyr Leu Ala Phe Leu Gly Asn Leu Val Ser Ala Gln Thr Val Phe Leu
       130                 135                 140
   Arg Pro Cys Leu Ser Met Ile Ala Ser His Phe Val Pro Pro Arg Val
   145                 150                 155                     160
   Ile Ile Lys Glu Gly Asp Val Asp Val Ser Asp Ser Asp Asp Glu Asp
                   165                 170                 175
   Asp Asn Leu Pro Ala Asn Phe Asp Thr Cys His Arg Ala Leu Gln Ile
               180                 185                 190
   Ile Ala Arg Tyr Val Pro Ser Thr Pro Trp Phe Leu Met Pro Ile Leu
           195                 200                 205
   Val Glu Lys Phe Pro Phe Val Arg Lys Ser Glu Arg Thr Leu Glu Cys
       210                 215                 220
   Tyr Val His Xaa Leu Leu Arg Ile Ser Val Tyr Phe Pro Thr Leu Arg
   225                 230                 235                     240
   His Glu Ile Leu Glu Leu Ile Ile Glu Lys Leu Leu Lys Leu Asp Val
   245 250 255
   Asn Ala Ser Arg Gln Gly Ile Glu Asp Ala Glu Glu Thr Ala Thr Gln
   260 265 270
   Thr Cys Gly Gly Thr Asp Ser Thr Glu Gly Leu Phe Asn Met Asp Glu
   275 280 285
   Asp Glu Glu Thr Glu His Glu Thr Lys Ala Gly Pro Glu Arg Leu Asp
   290 295 300
   Gln Met Val His Pro Val Ala Glu Arg Leu Asp Ile Leu Met Ser Leu
   305 310 315 320
   Val Leu Ser Tyr Met Lys Asp Val Cys Tyr Val Asp Gly Lys Val Asp
   325 330 335
   Asn Gly Lys Thr Lys Asp Leu Tyr Arg Asp Leu Ile Asn Ile Phe Asp
   340 345 350
   Lys Leu Leu Leu Pro Thr His Ala Ser Cys His Val Gln Phe Phe Met
   355 360 365
   Phe Tyr Leu Cys Ser Phe Lys Leu Gly Phe Ala Glu Ala Phe Leu Glu
   370 375 380
   His Leu Trp Lys Lys Leu Gln Asp Pro Ser Asn Pro Ala Ile Ile Arg
   385 390 395 400
   Gln Ala Ala Gly Asn Tyr Ile Gly Ser Phe Leu Ala Arg Ala Lys Phe
   405 410 415
   Ile Pro Leu Ile Thr Val Lys Ser Cys Leu Asp Leu Leu Val Asn Trp
   420 425 430
   Leu His Ile Tyr Leu Asn Asn Gln Asp Ser Gly Thr Lys Ala Phe Cys
   435 440 445
   Asp Val Ala Leu His Gly Pro Phe Tyr Ser Ala Cys Gln Ala Val Phe
   450 455 460
   Tyr Xaa Phe Val Phe Arg His Lys Gln Leu Leu Ser Gly Asn Leu Lys
   465 470 475 480
   Glu Gly Leu Gln Tyr Leu Gln Ser Leu Asn Phe Glu Arg Ile Val Met
   485 490 495
   Ser Gln Leu Asn Pro Leu Lys Ile Cys Leu Pro Ser Val Val Asn Phe
   500 505 510
   Phe Ala Ala Ile Thr Asn Lys Tyr Gln Leu Val Phe Cys Tyr Thr Ile
   515 520 525
   Ile Glu Arg Asn Asn Arg Gln Met Leu Pro Val Ile Arg Ser Thr Ala
   530 535 540
   Gly Gly Asp Ser Val Gln Ile Cys Thr Asn Pro Leu Asp Thr Phe Phe
   545 550 555 560
   Pro Phe Asp Pro Cys Val Leu Lys Arg Ser Lys Lys Phe Ile Asp Pro
   565 570 575
   Ile Tyr Gln Val Trp Glu Asp Met Ser Ala Glu Glu Leu Gln Glu Phe
   580 585 590
   Lys Lys Pro Met Lys Lys Asp Ile Val Glu Asp Glu Asp Asp Asp Phe
   595 600 605
   Leu Lys Gly Glu Val Pro Gln Asn Asp Thr Val Ile Gly Ile Thr Pro
       610                 615                 620
   Ser Ser Phe Asp Thr His Phe Arg Ser Pro Ser Ser Ser Val Gly Ser
   625                 630                 635                 640
   Pro Pro Val Leu Tyr Met Gln Pro Ser Pro Leu
                   645                 650

### SEQUENZPROTOKOLL

<110> Deutsches Krebsforschungszentrum
<120> RNA Polymerase I Transkriptionsfektor TIF-IA
<130> K 2804
<140> PCT/DE 00/00767
   <141> 2000-03-08
<150> DE 199 11 992.9
   <151> 1999-03-17
<160> 2
<170> PatentIn Ver. 2.1
<210> 1
   <211> 2040
   <212> DNA
   <213> Homo sapiens
<220>
   <221> CDS
   <222> (40) ... (1992)
<400> 1
   ggagcggccg cccaggtgcg gtcgcgttag ttcggccca atg gcg gca ccg ctg          54
                                             Met Ala Ala Pro Leu
                                              1               5
   ctt cac acg cgt ttg ccg gga gat gcg gcc gct tcg tcc tct gca gtt        102
   Leu His Thr Arg Leu Pro Gly Asp Ala Ala Ala Ser Ser Ser Ala Val
                   10                  15                  20
   aag aag ctg ggc gcg tcg agg act ggg att tca aat atg cgt gca tta        150
   Lys Lys Leu Gly Ala Ser Arg Thr Gly Ile Ser Asn Met Arg Ala Leu
               25                  30                  35
   gag aat gac ttt ttc aat tct ccc cca aga aaa act gtt cgg ttt ggt        198
   Glu Asn Asp Phe Phe Asn Ser Pro Pro Arg Lys Thr Val Arg Phe Gly
           40                  45                  50
   gga act gtg aca gaa gtc ttg ctg aag tac aaa aag ggt gaa aca aat        246
   Gly Thr Val Thr Glu Val Leu Leu Lys Tyr Lys Lys Gly Glu Thr Asn
       55                  60                  65
   gac ttt gag ttg ttg aag aac cag ctg tta gat cca gac ata aag gat        294
   Asp Phe Glu Leu Leu Lys Asn Gln Leu Leu Asp Pro Asp Ile Lys Asp
   70                  75                  80                  85
   gac cag atc atc aac tgg ctg cta gaa ttc cgt tct tct atc atg tac        342
   Asp Gln Ile Ile Asn Trp Leu Leu Glu Phe Arg Ser Ser Ile Met Tyr
                   90                  95                  100
   ttg aca aaa gac ttt gag caa ctt atc agt att ata tta aga ttg cct        390
   Leu Thr Lys Asp Phe Glu Gln Leu Ile Ser Ile Ile Leu Arg Leu Pro
               105                 110                 115
   tgg ttg aat aga agt caa aca gta gtg gaa gag tat ttg gct ttt ctt        438
   Trp Leu Asn Arg Ser Gln Thr Val Val Glu Glu Tyr Leu Ala Phe Leu
           120                 125                 130
   ggt aat ctt gta tca gca cag act gtt ttc ctc aga ccg tgt ctc agc      486
   Gly Asn Leu Val Ser Ala Gln Thr Val Phe Leu Arg Pro Cys Leu Ser
       135                 140                 145
   atg att gct tcc cat ttt gtg cct ccc cga gtg atc att aag gaa ggc      534
   Met Ile Ala Ser His Phe Val Pro Pro Arg Val Ile Ile Lys Glu Gly
   150                 155                 160                 165
   gat gta gat gtt tca gat tct gat gat gaa gat gat aat ctt cct gca      582
   Asp Val Asp Val Ser Asp Ser Asp Asp Glu Asp Asp Asn Leu Pro Ala
                   170                 175                 180
   aat ttt gac aca tgt cac aga gcc ttg caa ata ata gca aga tat gta      630
   Asn Phe Asp Thr Cys His Arg Ala Leu Gln Ile Ile Ala Arg Tyr Val
               185                 190                 195
   cca tcg aca ccg tgg ttt ctc atg cca ata ctg gtg gaa aaa ttt cca      678
   Pro Ser Thr Pro Trp Phe Leu Met Pro Ile Leu Val Glu Lys Phe Pro
           200                 205                 210
   ttt gtt cga aaa tca gag aga aca ctg gaa tgt tac gtt cat Nac tta      726
   Phe Val Arg Lys Ser Glu Arg Thr Leu Glu Cys Tyr Val His Xaa Leu
       215                 220                 225
   cta agg att agt gta tat ttt cca acc ttg agg cat gaa att ctg gag      774
   Leu Arg Ile Ser Val Tyr Phe Pro Thr Leu Arg His Glu Ile Leu Glu
   230                 235                 240                 245
   ctt att att gaa aaa cta ctc aag ttg gat gtg aat gca tcc cgg cag      822
   Leu Ile Ile Glu Lys Leu Leu Lys Leu Asp Val Asn Ala Ser Arg Gln
                   250                 255                 260
   ggt att gaa gat gct gaa gaa aca gca act caa act tgt ggt ggg aca      870
   Gly Ile Glu Asp Ala Glu Glu Thr Ala Thr Gln Thr Cys Gly Gly Thr
               265                 270                 275
   gat tcc acg gaa gga ttg ttt aat atg gat gaa gat gaa gaa act gaa      918
   Asp Ser Thr Glu Gly Leu Phe Asn Met Asp Glu Asp Glu Glu Thr Glu
           280                 285                 290
   cat gaa aca aag gct ggt cct gaa cgg ctc gac cag atg gtg cat cct      966
   His Glu Thr Lys Ala Gly Pro Glu Arg Leu Asp Gln Met Val His Pro
       295                 300                 305
   gta gcc gag cgc ctg gac atc ctg atg tct ttg gtt ttg tcc tac atg     1014
   Val Ala Glu Arg Leu Asp Ile Leu Met Ser Leu Val Leu Ser Tyr Met
   310                 315                 320                 325
   aag gat gtc tgc tat gta gat ggt aag gtt gat aac ggc aaa aca aag     1062
   Lys Asp Val Cys Tyr Val Asp Gly Lys Val Asp Asn Gly Lys Thr Lys
                   330                 335                 340
   gat cta tat cgc gac ctg ata aac atc ttt gac aaa ctc ctg ttg ccc     1110
   Asp Leu Tyr Arg Asp Leu Ile Asn Ile Phe Asp Lys Leu Leu Leu Pro
               345                 350                 355
   acc cat gcc tcc tgc cat gta cag ttt ttc atg ttt tac ctc tgt agt     1158
   Thr His Ala Ser Cys His Val Gln Phe Phe Met Phe Tyr Leu Cys Ser
           360                 365                 370
   ttc aaa ttg gga ttc gca gag gca ttt ttg gaa cat ctc tgg aaa aaa     1206
   Phe Lys Leu Gly Phe Ala Glu Ala Phe Leu Glu His Leu Trp Lys Lys
       375                 380                 385
   ttg cag gac cca agt aat cct gcc atc atc agg cag gct gct gga aat     1254
   Leu Gln Asp Pro Ser Asn Pro Ala Ile Ile Arg Gln Ala Ala Gly Asn
   390                 395                 400                 405
   tat att gga agc ttt ttg gca aga gct aaa ttt att cct ctt att act       1302
   Tyr Ile Gly Ser Phe Leu Ala Arg Ala Lys Phe Ile Pro Leu Ile Thr
                   410                 415                 420
   gta aaa tca tgc cta gat ctt ttg gtt aac tgg ctg cac ata tac ctt       1350
   Val Lys Ser Cys Leu Asp Leu Leu Val Asn Trp Leu His Ile Tyr Leu
               425                 430                 435
   aat aac cag gat tcg gga aca aag gca ttc tgc gat gtt gct ctc cat       1398
   Asn Asn Gln Asp Ser Gly Thr Lys Ala Phe Cys Asp Val Ala Leu His
           440                 445                 450
   gga cca ttt tac tca gcc tgc caa gct gtg ttc tac Ncc ttt gtt ttt       1446
   Gly Pro Phe Tyr Ser Ala Cys Gln Ala Val Phe Tyr Xaa Phe Val Phe
       455                 460                 465
   aga cac aag cag ctt ttg agc gga aac ctg aaa gaa ggt ttg cag tat       1494
   Arg His Lys Gln Leu Leu Ser Gly Asn Leu Lys Glu Gly Leu Gln Tyr
   470                 475                 480                 485
   ctt cag agt ctg aat ttt gag cgg ata gtg atg agc cag cta aat ccc       1542
   Leu Gln Ser Leu Asn Phe Glu Arg Ile Val Met Ser Gln Leu Asn Pro
                   490                 495                 500
   ctg aag att tgc ctg ccc tca gtg gtt aac ttt ttt gct gca atc aca       1590
   Leu Lys Ile Cys Leu Pro Ser Val Val Asn Phe Phe Ala Ala Ile Thr
               505                 510                 515
   aat aag tac cag ctc gtc ttc tgc tac acc atc att gag agg aac aat       1638
   Asn Lys Tyr Gln Leu Val Phe Cys Tyr Thr Ile Ile Glu Arg Asn Asn
           520                 525                 530
   cgc cag atg ctg cca gtc att agg agt acc gct gga gga gac tca gtg       1686
   Arg Gln Met Leu Pro Val Ile Arg Ser Thr Ala Gly Gly Asp Ser Val
       535                 540                 545
   cag atc tgc aca aac ccg ctg gac acc ttc ttc ccc ttt gat ccc tgt       1734
   Gln Ile Cys Thr Asn Pro Leu Asp Thr Phe Phe Pro Phe Asp Pro Cys
   550                 555                 560                 565
   gtg ctg aag agg tca aag aaa ttc att gat cct att tat cag gtg tgg       1782
   Val Leu Lys Arg Ser Lys Lys Phe Ile Asp Pro Ile Tyr Gln Val Trp
                   570                 575                 580
   gaa gac atg agt gct gaa gag cta cag gag ttc aag aaa ccc atg aaa       1830
   Glu Asp Met Ser Ala Glu Glu Leu Gln Glu Phe Lys Lys Pro Met Lys
               585                 590                 595
   aag gac ata gtg gaa gat gaa gat gat gac ttt ctg aaa ggc gaa gtg       1878
   Lys Asp Ile Val Glu Asp Glu Asp Asp Asp Phe Leu Lys Gly Glu Val
           600                 605                 610
   ccc cag aat gat acc gtg att ggg atc aca cca agc tcc ttt gac acg       1926
   Pro Gln Asn Asp Thr Val Ile Gly Ile Thr Pro Ser Ser Phe Asp Thr
       615                 620                 625
   cat ttc cga agt cct tca agt agt gtg ggc tcc cca ccc gtg ttg tac       1974
   His Phe Arg Ser Pro Ser Ser Ser Val Gly Ser Pro Pro Val Leu Tyr
   630                 635                 640                 645
   atg caa ccc agt ccc ctc tgacggcaga aatttgtgac tg agatgtgaca            2024
   Met Gln Pro Ser Pro Leu
                   650
   tttgggattc cccatc                                                     2040
<210> 2
   <211> 651
   <212> PRT
   <213> Homo sapiens
<400> 2
   Met Ala Ala Pro Leu Leu His Thr Arg Leu Pro Gly Asp Ala Ala Ala
   1 5 10 15
   Ser Ser Ser Ala Val Lys Lys Leu Gly Ala Ser Arg Thr Gly Ile Ser
   20 25 30
   Asn Met Arg Ala Leu Glu Asn Asp Phe Phe Asn Ser Pro Pro Arg Lys
   35 40 45
   Thr Val Arg Phe Gly Gly Thr Val Thr Glu Val Leu Leu Lys Tyr Lys
   50 55 60
   Lys Gly Glu Thr Asn Asp Phe Glu Leu Leu Lys Asn Gln Leu Leu Asp
   65 70 75 80
   Pro Asp Ile Lys Asp Asp Gln Ile Ile Asn Trp Leu Leu Glu Phe Arg
   85 90 95
   Ser Ser Ile Met Tyr Leu Thr Lys Asp Phe Glu Gln Leu Ile Ser Ile
   100 105 110
   Ile Leu Arg Leu Pro Trp Leu Asn Arg Ser Gln Thr Val Val Glu Glu
   115 120 125
   Tyr Leu Ala Phe Leu Gly Asn Leu Val Ser Ala Gln Thr Val Phe Leu
   130 135 140
   Arg Pro Cys Leu Ser Met Ile Ala Ser His Phe Val Pro Pro Arg Val
   145 150 155 160
   Ile Ile Lys Glu Gly Asp Val Asp Val Ser Asp Ser Asp Asp Glu Asp
   165 170 175
   Asp Asn Leu Pro Ala Asn Phe Asp Thr Cys His Arg Ala Leu Gln Ile
   180 185 190
   Ile Ala Arg Tyr Val Pro Ser Thr Pro Trp Phe Leu Met Pro Ile Leu
   195 200 205
   Val Glu Lys Phe Pro Phe Val Arg Lys Ser Glu Arg Thr Leu Glu Cys
   210 215 220
   Tyr Val His Xaa Leu Leu Arg Ile Ser Val Tyr Phe Pro Thr Leu Arg
   225 230 235 240
   His Glu Ile Leu Glu Leu Ile Ile Glu Lys Leu Leu Lys Leu Asp Val
   245 250 255
   Asn Ala Ser Arg Gln Gly Ile Glu Asp Ala Glu Glu Thr Ala Thr Gln
   260 265 270
   Thr Cys Gly Gly Thr Asp Ser Thr Glu Gly Leu Phe Asn Met Asp Glu
   275 280 285
   Asp Glu Glu Thr Glu His Glu Thr Lys Ala Gly Pro Glu Arg Leu Asp
   290 295 300
   Gln Met Val His Pro Val Ala Glu Arg Leu Asp Ile Leu Met Ser Leu
   305 310 315 320
   Val Leu Ser Tyr Met Lys Asp Val Cys Tyr Val Asp Gly Lys Val Asp
   325 330 335
   Asn Gly Lys Thr Lys Asp Leu Tyr Arg Asp Leu Ile Asn Ile Phe Asp
   340 345 350
   Lys Leu Leu Leu Pro Thr His Ala Ser Cys His Val Gln Phe Phe Met
   355 360 365
   Phe Tyr Leu Cys Ser Phe Lys Leu Gly Phe Ala Glu Ala Phe Leu Glu
   370 375 380
   His Leu Trp Lys Lys Leu Gln Asp Pro Ser Asn Pro Ala Ile Ile Arg
   385 390 395 400
   Gln Ala Ala Gly Asn Tyr Ile Gly Ser Phe Leu Ala Arg Ala Lys Phe
   405 410 415
   Ile Pro Leu Ile Thr Val Lys Ser Cys Leu Asp Leu Leu Val Asn Trp
   420 425 430
   Leu His Ile Tyr Leu Asn Asn Gln Asp Ser Gly Thr Lys Ala Phe Cys
   435 440 445
   Asp Val Ala Leu His Gly Pro Phe Tyr Ser Ala Cys Gln Ala Val Phe
   450 455 460
   Tyr Xaa Phe Val Phe Arg His Lys Gln Leu Leu Ser Gly Asn Leu Lys
   465 470 475 480
   Glu Gly Leu Gln Tyr Leu Gln Ser Leu Asn Phe Glu Arg Ile Val Met
   485 490 495
   Ser Gln Leu Asn Pro Leu Lys Ile Cys Leu Pro Ser Val Val Asn Phe
   500 505 510
   Phe Ala Ala Ile Thr Asn Lys Tyr Gln Leu Val Phe Cys Tyr Thr Ile
   515 520 525
   Ile Glu Arg Asn Asn Arg Gln Met Leu Pro Val Ile Arg Ser Thr Ala
   530 535 540
   Gly Gly Asp Ser Val Gln Ile Cys Thr Asn Pro Leu Asp Thr Phe Phe
   545 550 555 560
   Pro Phe Asp Pro Cys Val Leu Lys Arg Ser Lys Lys Phe Ile Asp Pro
   565 570 575
   Ile Tyr Gln Val Trp Glu Asp Met Ser Ala Glu Glu Leu Gln Glu Phe
   580 585 590
   Lys Lys Pro Met Lys Lys Asp Ile Val Glu Asp Glu Asp Asp Asp Phe
   595 600 605
   Leu Lys Gly Glu Val Pro Gln Asn Asp Thr Val Ile Gly Ile Thr Pro
   610 615 620
   Ser Ser Phe Asp Thr His Phe Arg Ser Pro Ser Ser Ser Val Gly Ser
   625 630 635 640
   Pro Pro Val Leu Tyr Met Gln Pro Ser Pro Leu
   645 650

## Beschreibung

Die vorliegende Erfindung betrifft einen RNA Polymerase I Transkriptionsfaktor TIF-IA und dazu verwandte Proteine, deren Konzentration und/oder Aktivität mit der Zellproliferationsrate korreliert sind, sowie diese Proteine codierende DNA-Sequenzen. Die vorliegende Erfindung betrifft ferner an RNA Polymerase I Transkriptionsfaktor TIF-IA bzw. dazu verwandte Proteine bindende Liganden, beispielsweise Antikörper, Antagonisten, sowie gegen die TIF-IA Expression gerichtete Antisense-RNAs bzw. Ribozyme. Schließlich betrifft die vorliegende Erfindung Arzneimittel und Diagnoseverfahren, bei denen die vorstehenden Verbindungen zur Anwendung kommen.

Die Synthese ribosomaler RNAs (rRNAs) durch RNA Polymerase I (Pol I) wird in Abhängigkeit von externen Signalen, wie z.B. Wachstumsfaktoren, Hormonen, Nährstoffen und Effektoren für Wachstum und Differenzierung äußerst effizient reguliert. Dementsprechend ist in Tumorzellen, die unkontrolliertes Zellwachstum aufweisen, die rRNA-Syntheserate drastisch erhöht (Grummt, I.:"Regulation of Mammalian Ribosomal Gene Transcription by RNA Polymerase I.", Progr. NAR & Mol. Biol. (1999) 62, 109-154). Die molekularen Mechanismen, die die Transkriptionsaktivität von Klasse I-Genen mit dem Zellwachstum verknüpfen, sind allerdings noch weitgehend unbekannt. Die Aufklärung der Prozesse, durch die extrazelluläre Signale in den Zellkern bzw. Nukleolus übertragen werden, um dort die Transkriptionsrate positiv oder negativ zu beeinflussen, erfordert daher die strukturelle und funktionelle Analyse der am Transkriptionsprozess beteiligten Proteinfaktoren, die Untersuchung deren Wechselwirkungen sowie die Identifizierung modifizierender Enzyme, beispielsweise Proteinkinasen und Proteinphosphatasen, die die Aktivität bestimmter Transkriptionsfakoren in Abhängigkeit von der zellulären Proliferationsrate regulieren.

Ein Faktor, der für die wachstumsabhängige Regulation der rDNA-Transkription verantwortlich ist, ist TIF-IA, dessen Konzentration und/oder Aktivität in Abhängigkeit von der Proliferationsrate der Zellen fluktuiert. TIF-IA wurde bereits 1983 funktionell identifiziert (Buttgereit et. al., Nucleic Acids Res. 13 (1985), 8165-8180) und in den folgenden Jahren biochemisch charakterisiert (Mahajan und Thompson, J.Biol.Chem.265 (1990), 16225-16233; Schnapp et al., Mol.Cell.Biol.13 (1993)., 6723-6732). Diese Untersuchungen lieferten folgende wichtige Befunde: (1) TIF-IA ist ein essentieller Initiationsfaktor für Pol I, der dem bakteriellen σ-Faktor funktionelle homolog zu sein scheint. Wie für den σ-Faktor gezeigt, ist auch TIF-IA mit der Initiationskompetenten Form der Pol I, dem Pol I "Holo-Enzym", assoziiert und wird nach der Initiationsreaktion von Pol I freigesetzt; (2) die Menge und/oder Aktivität von TIF-IA korreliert mit der Proliferationsrate der zellen, d.h. Extrakte aus Wachstumsarretierten Zellen sind transkriptionell inaktiv, sie können jedoch durch Zusatz von partiell gereinigtem TIF-IA komplementiert werden; und (3) die biochemische Aufreinigung von TIF-IA ergab, daß weniger als tausend TIF-IA-Moleküle selbst in exponentiell wachsenden zellen vorhanden sind.

WO-99/06549 A2 betrifft 5' ESTs für in Testis oder anderen Geweben exprimierte sekretierte Proteine.

Aufgrund der biologischen Eigenschaften von TIF-IA kann davon ausgegangen werden, daß dieser bei der Prävention und/oder Behandlung von Erkrankungen nützlich sein kann, bei der eine Stimulation der Zellproliferation durch eine Aktivierung der zelluläre rRNA-Synthese therapeutisch sinnvoll ist, beispielsweise zur Unterstützung der Gewebsregeneration nach Verletzungen oder Strahlentherapie. Andererseits kann auch die Möglichkeit der Inaktivierung von TIF-IA zur Proliferationshemmung bzw. zur Prävention und/oder Behandlung von Erkrankungen verwendet werden, die mit einer erhöhten Zellproliferation einhergehen, beispielsweise Tumorerkrankungen. Eine solche Inaktivierung kann auf verschiedenen Ebenen erfolgen, beispielsweise auf genetischer Ebene ("knock out", Hemmung der Translation durch Antisense-RNAs oder Ribozyme) oder Protein-Ebene (TIF-IA hemmende Liganen oder Antagonisten, Kinase/Phosphatase-Inihibotoren, Hemmung der der Bindung von TIF-IA an Pol I etc.). Voraussetzung für die vorstehend diskutierten therapeutischen Möglichkeiten ist allerdings, daß TIF-IA als Protein in ausreichenden Mengen und in möglichst reiner Form zur Verfügung steht bzw. das TIF-IA codierende Gen, was einerseits dessen rekombinante Herstellung ermöglicht, andererseits auch eine auf Gentherapie basierende Behandlung. Allerdings war bisher selbst die partielle Reinigung von TIF-IA äußerst schwierig, da, wie bereits ausgeführt, das Protein nur in sehr geringen Mengen in der Zelle vorhanden und vermutlich darüber hinaus sehr labil ist. Ausgehend von mehreren hundert Litern kultivierter Zellen konnte TIF-IA zwar über eine Kombination von neun chromatographischen Schritten gereinigt werden, wobei die TIF-IA-Aktivität mit einem 75 kDa Protein korreliert (Schnapp et al., supra), allerdings waren bisher weder die Reinheit noch die Menge an biochemisch gereinigtem TIF-IA ausreichend, um dieses therapeutisch einsetzen bzw. um partielle Aminosäuresequenzen bestimmen zu können, was die Entwicklung von Sonden für das TIF-IA codierende Gen erlauben würde.

Somit liegt der vorliegenden Erfindung das technische Problem zugrunde, (1) das TIF-IA-Protein in ausreichender Menge und reiner Form und (2) das TIF-IA codierende Gen bereitzustellen, was beispielsweise dessen rekombinante Herstellung gestattet.

Die Lösung dieses technischen Problems wird durch die Bereitstellung der in den Patentansprüchen gekennzeichneten Ausführungsformen erzielt.

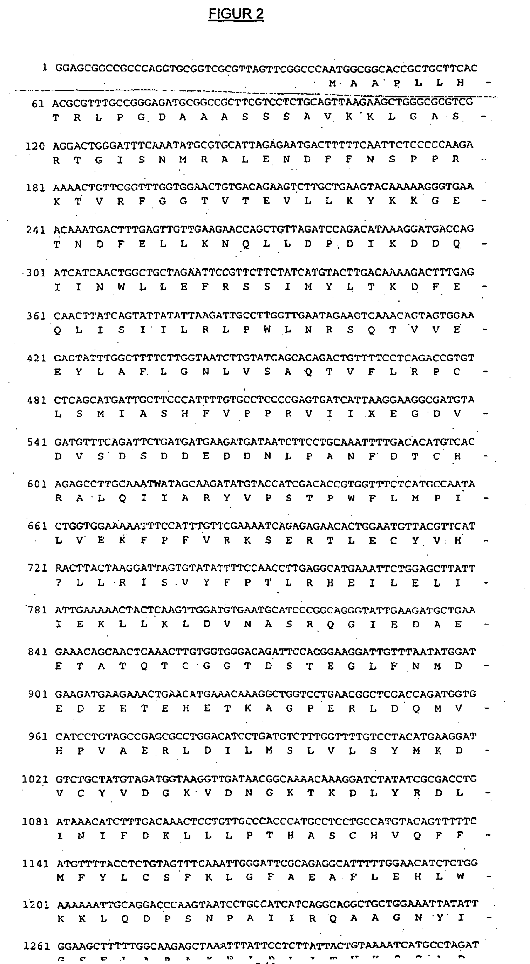

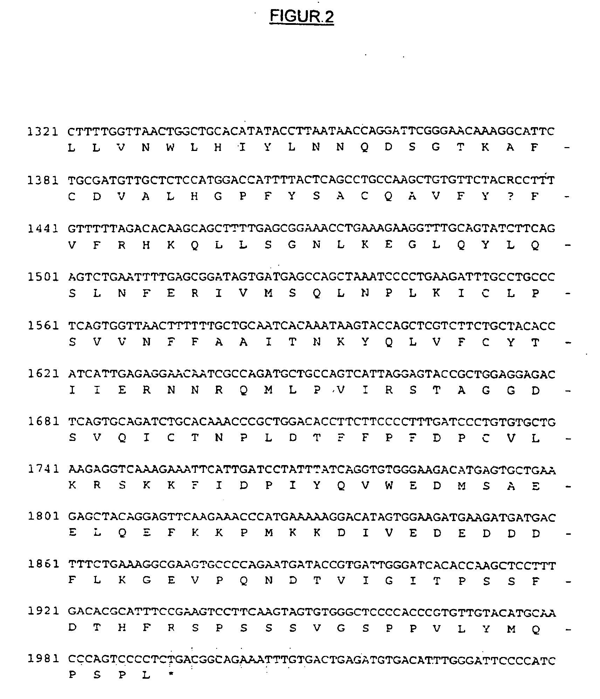

Gegenstand der vorliegenden Erfindung ist somit eine DNA-Sequenz, die einen RNA Polymerase I Transkriptionsfaktor TIF-IA mit der in Figur 2 gezeigten Aminosäuresequenz codiert, wobei in einer bevorzugten Ausführungsform die DNA-Sequenz die in Figur 2 gezeigte Nucleinsäuresequenz enthält.

Die vorliegende Erfindung beruht auf der Isolierung einer cDNA-Sequenz, die humanen TIF-IA codiert. Da es bisher nicht möglich war, TIF-IA in solchen Mengen und in solcher Reinheit bereitzustellen, daß - beispielsweise von einer partiellen Aminosäuresequenz ausgehend - Sonden für die erfolgreiche Clonierung des Gens entwickelt werden konnten, wurde in der vorliegenden Erfindung eine andere Strategie zur Bestimmung des TIF-IA codierenden Gens eingeschlagen. Dabei wurde in Genbanken nach DNA-Sequenzen gesucht, die Homologie zu Genen aufweisen, die für die Pol I Transkription in Hefe verantwortlich sind. Diese Vorgehensweise, die schließlich zur Isolierung eines TIF-IA codierenden Clons führte, wird in den nachstehenden Beispielen ausführlich beschrieben.

Die vorliegende Erfindung betrifft ferner eine DNA-Sequenz, die ein Protein mit den biologischen Eigenschaften eines RNA polymerase I Transkriptionsfaktors TIF-IA codiert, die sich von der DNA-Sequenz von Figur 2 in der Codonsequenz aufgrund der Degeneration des genetischen Codes unterscheidet, oder die ein Fragment der vorstehenden DNA-Sequenzen ist.

Der in dieser Anmeldung verwendete Begriff "hybridisieren" bezieht sich auf konventionelle Hybridisierungsbedingungen, vorzugsweise auf Hybridisierungsbedingungen, bei denen als Lösung 5xSSPE, 1% SDS, 1xDenhardts-Lösung verwendet wird und/oder die Hybridisierungstemperaturen zwischen 35°C und 70°C, vorzugweise bei 65°C liegen. Nach der Hybridisierung wird vorzugsweise zuerst mit 2xSSC, 1% SDS und danach mit 0,2xSSC bei Temperaturen zwischen 35°C und 70°C, vorzugsweise bei 65°C gewaschen (zur Definition von SSPE,SSC und Denhardts-Lösung siehe Sambrook et al., Molecular Cloning: A Laboratory Manual, 2. Ausgabe, Cold Spring Harbor Laboratory Press, Cold Spring Harbor NY (1989)). Besonders bevorzugt sind stringente Hybridisierungsbedingungen, wie sie beispielsweise in Sambrook et al ., supra, beschrieben sind.

Die in der vorliegenden Anmeldung verwendeten Begriffe "Varianten" oder "Fragment" umfassen DNA-Sequenzen, die sich gegenüber den in Figur 2 angegebenen Sequenzen durch Deletion(en), Insertion(en), Austausch(e) und/oder andere im Stand der Technik bekannte Modifikationen unterscheiden bzw. ein Fragment des ursprünglichen Nucleinsäuremoleküls umfassen, wobei das durch diese DNA-Sequenzen codierte Protein noch die biologischen Eigenschaften von TIF-IA aufweist und in Säugern biologisch aktiv ist. Dazu zählen auch Allelvarianten. Verfahren zur Erzeugung der vorstehenden Änderungen in der Nucleinsäuresequenz sind dem Fachmann bekannt und in Standardwerken der Molekularbiologie beschrieben, beispielsweise in Sambrook et al., supra. Der Fachmann ist auch in der Lage, zu bestimmen, ob ein von einer so veränderten Nucleinsäuresequenz codiertes Protein noch über die biologischen Eigenschaften von TIF-IA verfügt.

Durch die Erniedrigung oder Hemmung der Expression der vorstehend beschriebenen DNA-Sequenzen, kann die Synthese von TIF-IA bzw. verwandten Proteinen verringert oder eliminiert werden, was beispielsweise bei den in der Einleitung beschriebenen Krankheitszuständen wünschenswert ist. Daher betrifft eine weitere bevorzugte Ausführungsform der vorliegenden Erfindung eine Antisense-RNA, die dadurch gekennzeichnet ist, daß sie zu den vorstehenden DNA-Sequenzen komplementär ist und die Synthese des von diesen DNA-Sequenzen codierten TIF-IA verringern oder hemmen kann und ein Ribozym, das dadurch gekennzeichnet ist, daß es zu den vorstehenden DNA-Sequenzen komplementär ist und an die von diesen DNA-Sequenzen transkribierte RNA spezifisch binden und diese spalten kann, wodurch die Synthese des von diesen DNA-Sequenzen codierten TIF-IA ebenfalls verringert oder gehemmt wird. Vorzugsweise sind diese Antisense-RNAs und Ribozyme zu einer codierenden Region der mRNA komplementär. Der Fachmann ist in der Lage, ausgehend von den offenbarten DNA-Sequenzen, geeignete Antisense-RNAs herzustellen und anzuwenden. Geeignete Vorgehensweisen sind beispielsweise in EB-B1 0 223 399 oder EP-A1 0 458 beschrieben. Ribozyme sind RNA-Enzyme und bestehen aus einem einzelnen RNA-Strang. Diese können andere RNAs intermolekular spalten, beispielsweise die von den erfindungsgemäßen DNA-Sequenzen transkribierten mRNAs. Diese Ribozyme müssen prinzipiell über zwei Domänen verfügen, (1) eine katalytische Domäne und, (2) eine Domäne, die zu der Ziel-RNA komplementär ist und an diese binden kann, was die Voraussetzung für eine Spaltung der Ziel-RNA ist. Ausgehend von in der Literatur beschriebenen Vorgehensweisen ist es inzwischen möglich, spezifische Ribozyme zu konstruieren, die eine gewünschte RNA an einer bestimmten, vorgewählten Stelle schneiden (siehe beispielsweise Tanner et al., in: Antisense Research and Applications, CRC Press, Inc. (1993), 415-426). Vorzugsweise weisen die erfindungsgemäßen Antisense-RNAs bzw. Ribozyme einen Bereich auf, der zu der Ziel-DNA über eine Länge von 12 Nucleotiden, bevorzugt 15 Nucleotiden, stärker bevorzugt 20 Nucleotiden komplementär ist.

Die erfindungsgemäßen DNA-Sequenzen bzw. die die vorstehend beschriebenen Antisense-RNAs oder Ribozyme codierenden DNAs können auch in einen Vektor bzw. Expressionsvektor inseriert werden. Somit umfaßt die vorliegende Erfindung auch diese DNA-Sequenzen enthaltende Vektoren bzw. Expressionsvektoren. Die Bezeichnung "Vektor" bezieht sich auf ein Plasmid (pUC18, pBR322, pBlueScript etc.), auf ein Virus oder ein anderes geeignetes Vehikel. In einer bevorzugten Ausführungsform ist das erfindungsgemäße DNA-Molekül im Vektor mit regulatorischen Elementen funktionell verknüpft, die dessen Expression in prokaryotischen oder eukaryotischen Wirtszellen erlauben. Solche Vektoren enthaltend neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryoten, beispielsweise E.coli, zählen der lac-,trp-Promotor oder T7-Promotor, und für die Expression in Eukaryoten der AOX1- oder GAL1-Promotor in Hefe und der CMV-,SV40-,RVS-40-Promotor, CMV- oder SV40-Enhancer für die Expression in tierischen Zellen. Weitere Beispiele für geeignete Promotoren sind der Metallothionein I- und der Polyhedrin-Promotor. Zu geeigneten Expressionsvektoren für E.coli zählen beispielsweise pGEMEX, pUC-Derivate, pGEX-2T, pET3b und pQE-8, wobei letzterer bevorzugt ist. Zu den für die Expression in Hefe geeigneten Vektoren zählen pY100 und Ycpad1, für die Expression in Säugerzellen pMSXND, pKCR, pEFBOS, cDM8 und pCEV4. Zu den erfindungsgemäßen Expressionsvektoren zählen auch von Baculovirus abgeleitete Vektoren für die Expression in Insektenzellen, beispielsweise pAcSGHisNT-A.

Allgemeine, auf dem Fachgebiet bekannte Verfahren können zur Konstruktion von Expressionsvektoren, die die erfindungsgemäßen DNA-Sequenzen und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind. Die erfindungsgemäßen DNA-Sequenzen können auch in Verbindung mit einer für ein anderes Protein bzw. Peptid codierenden DNA inseriert werden, so daß die erfindungsgemäßen DNA-Sequenzen beispielsweise in Form eines Fusionsproteins exprimiert werden können.

Die vorliegende Erfindung betrifft auch die vorstehend beschriebenen Vektoren enthaltende Wirtszellen. Zu diesen Wirtszellen zählen Bakterien (beispielsweise die E.coli-Stämme HB101, DH1, x1776, JM101, JM109, BL21 und SG13009), Hefe, vorzugsweise S. cerevisiae, Insektenzellen, vorzugsweise sf9-Zellen, und Tierzellen, vorzugsweise Säugerzellen. Bevorzugte Säugerzellen sind CHO-, VERO-, BHK-, HeLa-, COS-, MDCK, 293- und WI38-Zellen. Verfahren zur Transformation dieser Wirtszellen, zur phänotypischen Selektion von Transformanten und zur Expression der erfindungsgemäßen DNA-Moleküle unter Verwendung der vorstehend beschriebenen Vektoren sind auf dem Fachgebiet bekannt.

Die vorliegende Erfindung betrifft ferner einen von den erfindungsgemäßen DNA-Sequenzen codierten RNA Polymerase I Transkriptionsfaktor TIF-IA bzw. Proteine mit dessen biologischer Aktivität sowie Verfahren zu deren rekombinanten Herstellung unter Verwendung der erfindungsgemäßen Expressionsvektoren. Das erfindungsgemäße Verfahren umfaßt die Kultivierung der vorstehend beschriebenen Wirtszellen unter Bedingungen, die die Expression des Proteins (bzw. Fusionsproteins) erlauben (vorzugsweise stabile Expression), und die Gewinnung des Proteins aus der Kultur oder aus den Wirtszellen. Dem Fachmann sind Bedingungen bekannt, transformierte bzw. transfizierte Wirtszelle zu kultivieren. Geeignete Reinigungsverfahren (beispielsweise präparative Chromatographie, Affinitätschromatographie, beispielsweise Immunoaffinitätschromatographie, HPLC etc.) sind ebenfalls allgemein bekannt.

Nützlich sind auch Liganden gegen die vorstehend beschriebenen erfindungsgemäßen Proteine (TIF-IA oder verwandte Proteine). Diese Liganden können beispielsweise in diagnostischen Assays verwendet werden oder für therapeutische Zwecke, wobei es nach Anlagerung an das Zielmolekül, d.h. TIF-IA, zu dessen Hemmung kommt, d.h. es kann nicht mehr an sein Ziel, Pol I, binden und diese somit nicht mehr aktivieren. Verfahren zur Isolierung bzw. Synthese solcher Liganden sind dem Fachmann bekannt. Beispielsweise können möglicherweise nützliche aktivitätshemmende Verbindungen in Extrakten von natürlichen Produkten als Ausgangsmaterial gescreent werden. Solche Extrakte können beispielsweise aus Pilzen, Actinomyceten, Algen, Insekten, Protozoen, Pflanzen und Bakterien stammen.

Erfindungsgemäß handelt es sich bei dem Liganden um einen Antikörper oder ein Fragment davon. Diese Antikörper können monoclonale, polyclonale oder synthetische Antikörper sein oder Fragmente davon. In diesem Zusammenhang bedeutet der Begriff "Fragment" alle Teile des monoclonalen Antikörpers (z.B. Fab-, Fv- oder "single chain Fv"-Fragmente), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörpern um monoclonale Antikörper. Die erfindungsgemäßen Antikörper können gemäß Standardverfahren hergestellt werden, wobei vorzugsweise der von den erfindungsgemäßen DNA-Sequenzen codierte TIF-IA oder ein synthetisches Fragment davon als Immunogen dienen. Verfahren zur Gewinnung monoclonaler Antikörper sind dem Fachmann bekannt.

In einer besonders bevorzugten Ausführungsform ist der genannte monoclonale Antikörper ein aus einem Tier (z.B. Maus) stammender Antikörper, ein humanisierter Antikörper oder ein chimärer Antikörper oder ein Fragment davon. Chimäre, menschlichen Antikörper ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 10029, und Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z.B. von der Maus) stammen (mit der Bezeichnung Donor-Immunglobulin). Die (der) konstante(n) Bereich(e) stammt (stammen), falls vorhanden, auch im wesentlichen von einem menschlichen Immunglobulin. Bei der Verabreichung an menschliche Patienten bieten humanisierte (sowie die menschlichen) Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörperantwort gegen einen solchen injizierten Antikörper geringer ausfallen als gegen einen vollständig fremden Maus-Antikörper oder einen partiell fremden chimären Antikörper; (b) da der Effektorbereich des humanisierten Antikörpers menschlich ist, interagiert er besser mit anderen Teilen des menschlichen Immunsystems, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

Die erfindungsgemäßen Antikörper können beispielsweise auch zur Immunpräzipitation des erfindungsgemäßen TIF-IA, zur Isolierung verwandter Proteine aus cDNA-Expressionsbanken oder für diagnostische Zwecke (siehe unten) verwendet werden. Die vorliegende Erfindung betrifft auch ein Hybridom, das den vorstehend beschriebenen monoclonalen Antikörper erzeugt.

Erwähnt seien auch Antagonisten für TIF-IA bzw. damit verwandte Proteine. Diese erlauben in vivo ebenfalls eine Hemmung der rRNA-Transkriptionsrate. Zu den möglichen Antagonisten zählen Peptide, die dadurch gewonnen werden können, daß beispielsweise Oligopeptide mit nach dem Zufallsprinzip erzeugten Sequenzen gescreent werden, um so TIF-IA Inhibitoren auffinden zu können. Solche Peptide können direkt als Wirkstoffe verwendet werden, oder um die Orientierung oder Lage einer funktionellen Gruppe aufzufinden, die TIF-IA Aktivität hemmen kann, was wiederum zum Entwurf und dem Austesten eines inhibitorischen kleinen Moleküls führt, oder wobei das Peptid das Rückgrat für chemische Modifizierung wird, die dessen pharmakologischen Verwendungsmöglichkeiten steigern. Bei dem Peptid kann es sich um strukturelle Imitatoren handeln, es können aber auch Molekülmodulierungsprogramme verwendet werden, um Imitatoren - basierend auf der charakteristischen Sekundär- und/oder Tertiärstruktur von TIF-IA - zu entwerfen. Solche strukturellen Imitatoren können dann in vivo als TIF-IA Inhibitoren verwendet werden. Zu den als Antagonisten wirksamen Verbindungen zählen auch beispielsweise inaktive TIF-IA Moleküle. Wenn diese in ausreichend hoher Konzentration exprimiert bzw. verabreicht werden, führt dies zur Verarmung an notwendigen aktiven TIF-IA Molekülen und dadurch fungieren diese als TIF-IA Antagonisten.

Die vorliegende Erfindung erlaubt auch die Durchführung therapeutischer Maßnahmen bei den vorstehend diskutierten Störungen der Zellproliferation bzw. den damit in Zusammenhang stehenden Erkrankungen, d.h. die vorstehend beschriebenen, erfindungsgemäßen DNA-Sequenzen, Antisense-RNAs, Ribozyme und/oder Antikörper können auch zur Herstellung eines Arzneimittels, beispielsweise zur Kontrolle der Expression des TIF-IA (oder des damit verwandten Proteins) oder zum Austausch einer mutierten Form des Gens gegen eine funktionelle Form verwendet werden, somit beispielsweise einerseits zur Herstellung eines Arzneimittels zur Prävention oder der Behandlung von Erkrankungen, die mit einer erhöhten Zellproliferation assoziiert sind oder zur Hemmung der Zellproliferation, insbesondere Tumorerkrankungen, andererseits zur Herstellung eines Arzneimittels zur Prävention oder der Behandlung von Erkrankungen, die mit einer verringerten Zellproliferation assoziiert sind oder zur Stimulation der Zellproliferation, beispielsweise zur Förderung der Geweberegenation. Beispielsweise kann der erfindungsgemäße TIF-IA in Säuger, insbesondere den Menschen, durch übliche Maßnahmen eingebracht werden. Hierzu kann es günstig sein, das Protein an ein vom jeweiligen Körper nicht als fremd angesehenes Protein, z.B. Transferrin oder Rinderserumalbumin (BSA), zu koppeln. Auch kann eine erfindungsgemäße DNA-Sequenz, Antisense-RNA oder Ribozym in Säuger, insbesondere den Menschen, eingebracht und exprimiert werden. Mit einem erfindungsgemäßen Liganden, beispielsweise einem monoclonalen Antikörper, kann die Expression von TIF-IA bzw. der verwandten Proteine kontrolliert und reguliert werden. Antagonisten für TIF-IA können ebenfalls dazu verwendet werden, um so die Wirkung von aktivem TIF-IA abzuschwächen oder ganz zu blockieren.

Somit betrifft die vorliegende Erfindung auch ein Arzneimittel, das die vorstehend beschriebenen DNA-Sequenzen, Antisense-RNA, das Ribozym, den Expressionsvektor, das erfindungsgemäße Protein und/oder den Antikörper enthält. Dieses Arzneimittel enthält gegebenenfalls zusätzlich einen pharmazeutisch verträglichen Träger, Geeignete Träger und die Formulierung derartiger Arzneimittel sind dem Fachmann bekannt. Zu geeigneten Trägern zählen beispielsweise Phosphatgepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, dem Stadium der Erkrankung, der Art der Verabreichung etc.

Vorzugsweise werden die vorstehend beschriebenen DNA-Sequenzen in einen für die Gentherapie geeigneten Vektor inseriert, beispielsweise unter Kontrolle eines gewebespezifischen Promotors, und in die Zellen eingeschleust. In einer bevorzugten Ausführungsform ist der die vorstehend beschriebenen DNA-Sequenzen enthaltende Vektor ein Virus, beispielsweise ein Adenovirus, Vaccinia-Virus oder Adenovirus. Besonders bevorzugt sind Retroviren. Beispiele für geeignete Retroviren sind MoMuLV, HaMuSV, MuMTV, RSV oder GaLV. Für Zwecke der Gentherapie können die erfindungsgemäßen DNA-Sequenzen auch in Form von kolloidalen Dispersionen zu den Zielzellen transportiert werden. Dazu zählen beispielsweise Liposomen oder Lipoplexe (Mannino et al., Biotechniques 6 (1988), 682). Diese Gentherapie kann sowohl für die Erhöhung der Proliferationsrate der Zellen (beispielsweise mit einem Vektor, der das Gen für aktiven TIF-IA enthält) als auch für eine Verringerung der Proliferationsrate (mit einem Vektor, der ein Gen für ein Ribozym, eine Antisense-RNA oder eine inaktive Form von TIF-IA ("knock-out") enthält) Anwendung finden.

Die vorliegende Erfindung ermöglicht es auch, Störungen der TIF-IA Expression bzw. Zellproliferation auf genetischer Ebene zu untersuchen. Mit einer erfindungsgemäßen DNA-Sequenz bzw. davon abgeleiteten Sonden oder Primern kann in Säugern, insbesondere dem Menschen, festgestellt werden, ob sie ein verändertes TIF-IA Gen enthalten, das zu einer mutierten Form des Proteins führt, die nicht länger biologisch aktiv ist, oder ob beispielsweise TIF-IA zu gering oder zu stark exprimiert wird. Dazu kann der Fachmann übliche Verfahren, wie Reverse Transkription, PCR, LCR, Hybridisierung und Sequenzierung durchführen. Auch die erfindungsgemäßen Liganden, beispielsweise die Antikörper oder Fragmente davon, eignen sich für die Diagnostik, d.h. beispielsweise zum Nachweis des Vorhandenseins und/oder der Konzentration des TIF-IA, einer verkürzten oder verlängerten Form dieses Proteins etc., in einer Probe. Die Antikörper können beispielsweise in Immunoassays in Flüssigphase oder an einen festen Träger gebunden werden. Dabei können die Antikörper auf verschiedene Art und Weise markiert sein. Geeignete Marker und Markierungsverfahren sind auf dem Fachgebiet bekannt. Beispiele für Immunassays sind ELISA und RIA.

Somit betrifft die vorliegende Erfindung auch ein Diagnoseverfahren zum Nachweis einer gestörten TIF-IA Expression, bei dem man eine Probe mit der erfindungsgemäßen DNA-Sequenz oder dem erfindungsgemäßen Liganden, beispielsweise einem monoclonalen Antikörper oder einem Fragment davon in Berührung bringt und sodann direkt oder indirekt bestimmt, ob sich die Konzentration, Länge und/oder Sequenz des RNA Polymerase I Transkriptionsfaktors TIF-IA oder der diesen codierenden mRNA im Vergleich zu einer Kontrollprobe unterscheiden. Die für dieses Diagnoseverfahren verwendbaren Sonden umfassen auch auf den erfindungsgemäßen DNA-Sequenzen basierende Primer, beispielsweise für eine PCR.

Schließlich betrifft die vorliegende Erfindung einen diagnostischer Kit zur Durchführung des vorstehend beschriebenen Diagnoseverfahrens, der eine erfindungsgemäße DNA-Sequenz oder den vorstehend beschriebenen, erfindungsgemäßen Liganden, beispielsweise einen monoclonalen Antikörper oder das Fragment davon, enthält. Je nach Ausgestaltung des diagnostischen Kits können die DNA-Sequenz bzw. der Ligand immobilisiert sein.

### Kurze Beschreibung der Figuren:

**Fig. 1****:** Vergleich der Aminosäuresequenzen von rrn3p aus S.cerevisiae (rrn3p) und homologen Proteinen aus S. pombe (pombe), C. elegans (cec36e8) und Arabidopsis thaliana (ATAC)
**Fig. 2****:** humane cDNA-Sequenz TIF-IA und abgeleitete Aminosäuresequenz

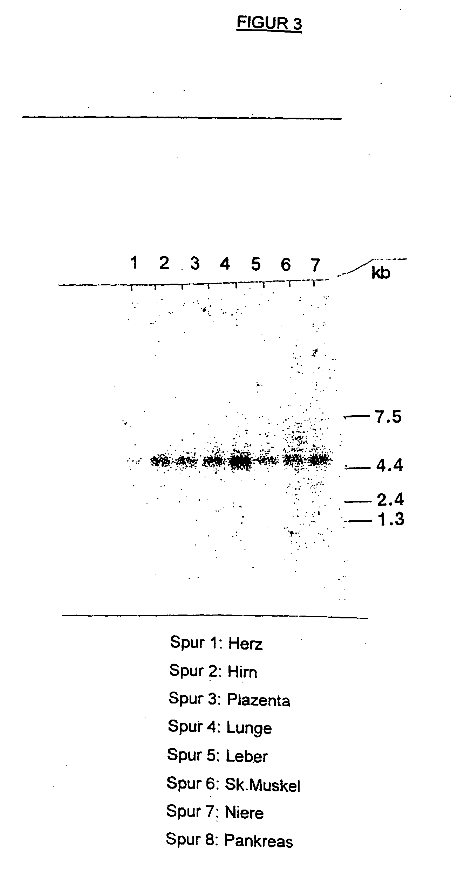

**Fig. 3****:** Northern-Blot-Analyse
   Zum Nachweis von TIF-IA-Sequenzen wurden aus verschiedenen Geweben extrahierte RNAs (Clontech: 7760-1) mit radioaktiv markierter TIF-IA cDNA (Clon pBS-hTIF-IA) hybridisiert und anschließend eine Autoradiographie erstellt.

Die folgenden Beispiele erläutern die Erfindung.

### Beispiel 1: Identifizierung einer TIF-IA cDNA-Sequenz

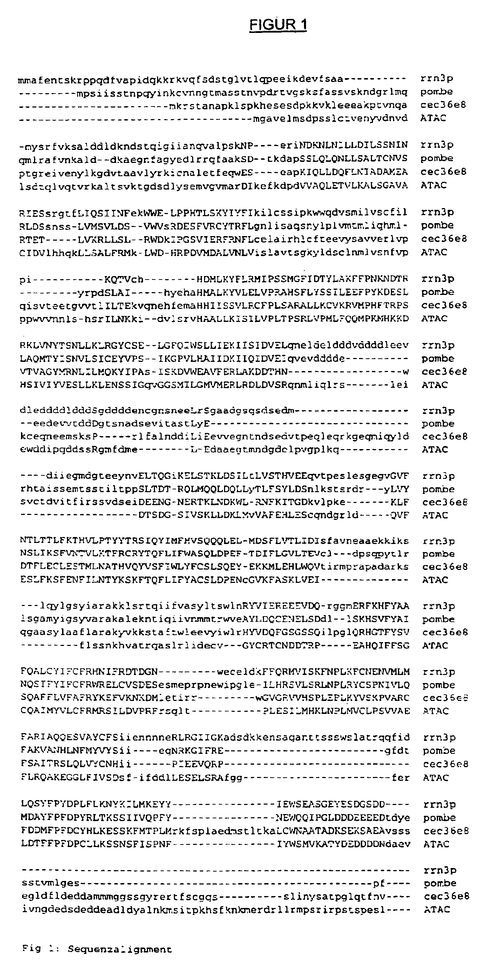

Es sind Genprodukte von Hefegenen bekannt, die für die Pol I Transkription in S. cerevisiae erforderlich sind. Bei einem der Genprodukte, rrn3p, handelt es sich um ein 72 kDa Protein, das mit der drittgrößten Untereinheit von Pol I (RPA49) assoziiert ist. Dieses scheint in stationären Hefekulturen entweder nicht vorhanden oder transkriptionell inaktiv zu sein. Solche Eigenschaften charakterisieren auch TIF-IA. Der Anmelder hat daher vermutet, daß es sich bei rrn3p und TIF-IA um funktionell homologe Proteine handeln könnte. Er hat daher eine umfangreiche Suche in verschiedenen Datenbanken durchgeführt, um Informationen über die mögliche Existenz eines rrn3p-homologen Proteins in anderen Organismen zu erhalten. Mit Hilfe des "BLAST"-Programms hat er Homologien zwischen rrn3p aus S.cerevisiae und einem Genprodukt aus S. pombe (Swissprot ID YAQA_SCHPO), einem Genprodukt aus Arabidopsis thaliana( PID:g3132470) und einem Genprodukt aus C. elegans (PID:g3924707) identifiziert. Die Vergleiche der Aminosäuresequenzen sind in Fig. 1 gezeigt. Diese wurden nach dem Algorithmus von Vingron und Argos, J.Mol.Biol.218 (1991), 33-43, durchgeführt und zeigen in Großbuchstaben die konservierten Bereiche. Hieraus konnte folgende Konsensussequenz für den C-terminalen Bereich abgeleitet werden:

Mit dieser "Core"-Sequenz hat der Anmelder humane ESTs und genomische Sequenzbanken durchsucht. Dabei wurden drei Homologie-Bereiche in der genomischen Sequenz eines "BAC"-Clons (CIT987SK-270G1, "Genbank"-Zugangsnummer AF001549) gefunden. Es wurde in einem Bereich von ca. 10.000 Basen, der die gefundenen Homologien enthält, mit dem Programm "GENSCAN" eine Exonvorhersage berechnet, die die drei gefundenen homologen Bereiche in der konservierten Region als codierende Sequenzen bestätigte. Ausgehend von dieser Sequenz wurde das 5'Ende des zu rrn3p homologen Gens mit einem 5'RACE aus einer humanen fötalen cDNA-Bank bestimmt. Durch "gene walking", gezielte PCR-Strategien, Hybridisierungstechniken, 3'RACE, Sequenzierung und Zusammenfügung der einzelnen Genabschnitte wurde letztlich ein cDNA-Clon erhalten, der für TIF-IA mit einem Molekulargewicht von etwa 75 kDa codiert. Die Nucleinsäuresequenz und die davon abgeleitete Aminosäuresequenz sind in Fig. 2 dargestellt. Dieser cDNA-Clon wurde mit pBS-hTIF-IA bezeichnet. Er wurde bei der DSMZ (Deutsche Sammlung von Mikroorganismen und Zellkulturen) unter DSM 12707 am 25.2.1999 hinterlegt.

### Beispiel 2: Nachweis von TIF-IA-Sequenzen in Geweben

Käufliche Filter (Clontech: 7760-1), die RNAs von Herz, Hirn, Plazenta, Lunge, Leber, Skelettmuskel, Niere und Pankreas enthalten, wurden einer Hybridisierung mit [α³²P] dCTP markierter pBS-hTIF-IA cDNA von Beispiel 1 unterworfen. Die Hybridisierung erfolgte bei 65° C in 5 x SSPE, 1 % SDS, 1 x Denhardts-Lösung. Nach der Hybridisierung wurde zuerst mit 2 x SSC, 1 % SDS und danach mit 0,2 x SSC bei 65° C gewaschen.

Es zeigte sich, daß TIF-IA-Sequenzen in den verschiedensten Geweben unterschiedlich stark exprimiert werden.

### Beispiel 3: Herstellung und Reinigung eines erfindungsgemäßen RNA Polymerase I Transkriptionsfaktors TIF-IA.

Die DNA von Fig. 2 wurde mit BAMHI-Linkern versehen, mit BamHI nachgespalten und in den mit BamHI gespaltenen Expressionsvektors pQE-8 (Qiagen) inseriert. Es wurde das Expressionsplasmid pQE-8/TIF-IA erhalten. Ein solches kodiert für ein Fusionsprotein aus 6 Histidin-Resten (N-Terminuspartner) und dem erfindungsgemäßen Tif-IA von Fig. 2 (C-Terminuspartner). pQE-8/TIF-IA wurde zur Transformation von E.coli SG 13009(vgl. Gottesman, S. et al., J. Bacteriol. 148, (1981), 265-273) verwendet. Die Bakterien wurden in einem LB-Medium mit 100µg/ml Ampicillin und 25µg/ml Kanamycin kultiviert und 4 h mit 60µM Isopropyl-ß-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Qiagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein erfindungsgemäßes (Fusions)protein in hochreiner Form hergestellt werden kann.

## Patentansprüche

1. DNA-Sequenz, kodierend für einen RNA Polymerase I Transkriptionsfaktor TIF-IA mit der in Fig. 2 gezeigten Aminosäuresequenz.

2. DNA-Sequenz nach Anspruch 1, wobei sie die in Fig. 2 gezeigte Nucleinsäuresequenz umfaßt.

3. DNA-Sequenz, kodierend für ein Protein mit der biologischen Eigenschaft des RNA Polymerase I Transkriptionsfaktors TIF-IA als essentieller Initiationsfaktor für Pol I, wobei die DNA-Sequenz
(a) sich von der DNA-Sequenz von Anspruch 2 in der Codonsequenz aufgrund der Degeneration des genetischen Codes unterscheidet; oder
(b) ein Fragment der DNA-Sequenz von Anspruch 2 oder 3(a) ist.

4. Ribozym, wobei es zu der DNA-Sequenz nach einem der Ansprüche 1 bis 3 komplementär ist und an die von dieser DNA-Sequenz transkribierte RNA spezifisch binden und diese spalten kann, wodurch die Synthese des von dieser DNA-Sequenz codierten Proteins verringert oder gehemmt wird.

5. Antisense-RNA, wobei sie zu der DNA-Sequenz nach einem der Ansprüche 1 bis 3 komplementär ist und an die von dieser DNA-Sequenz transkribierte RNA spezifisch binden kann, wodurch die Synthese des von dieser DNA-Sequenz codierten Proteins verringert oder gehemmt wird.

6. Expressionsvektor, wobei er die DNA-Sequenz nach einem der Ansprüche 1 bis 3 enthält oder für das Ribozym nach Anspruch 4 oder die Antisense-RNA nach Anspruch 5 kodiert.

7. Wirtszelle, enthaltend den Expressionsvektor nach Anspruch 6.

8. Isolierter RNA Polymerase I Transkriptionsfaktor TIF-IA oder isoliertes Protein mit dessen biologischer Aktivität als essentieller Initiationsfaktor für Pol I, das (der) durch die DNA-Sequenz nach einem der Ansprüche 1 bis 3 kodiert wird.

9. Verfahren zur Herstellung eines RNA Polymerase I Transkriptionsfaktors TIF-IA oder eines Proteins mit dessen biologischer Aktivität nach Anspruch 8, das die Züchtung der wirtszelle nach Anspruch 7 unter geeigneten Bedingungen und die Gewinnung des Proteins aus der Zelle oder dem Zuchtmedium umfaßt.

10. Antikörper oder ein Fragment des Antikörpers, der (das) an den RNA Polymerase I Transkriptionsfaktor TIF-IA oder ein Protein mit dessen biologischer Aktivität nach Anspruch 8 bindet.

11. Arzeimittel, enthaltend die DNA-Sequenz nach einem der Ansprüche 1 bis 3, den Expressionsvektor nach Anspruch 6, den RNA Polymerase I Transkriptionsfaktor TIF-IA oder ein Protein mit dessen biologischer Aktivität nach Anspruch 8.

12. Arzneimittel, enthaltend das Ribozym nach Anspruch 4, die Antisense-RNA nach Anspruch 5, oder den Antikörper oder das Fragment davon nach Anspruch 10.

13. Diagnoseverfahren zum Nachweis einer eventuell gestörten TIF-IA Expression, bei dem man eine Probe mit der DNA-Sequenz nach einem der Ansprüche 1 bis 3 oder dem Antikörper oder das Fragment davon nach Anspruch 10 in Berührung bringt und sodann direkt oder indirekt bestimmt, ob sich die Konzentration, Länge und/oder Sequenz des RNA Polymerase I Transkriptionsfaktors TIF-IA oder der diesen codierenden mRNA im Vergleich zu einer Kontrollprobe unterscheiden.

14. Diagnostischer Kit zur Durchführung des Verfahrens nach Anspruch 13, der die DNA-Sequenz nach einem der Ansprüche 1 bis 3 und/oder den Antikörper oder das Fragment davon nach Anspruch 10 enthält.

## Claims

1. A DNA sequence coding for an RNA polymerase I transcription factor TIF-IA having the amino acid sequence shown in fig. 2.

2. The DNA sequence according to claim 1, which comprises the nucleic acid sequence shown in fig. 2.

3. The DNA sequence coding for a protein having the biological property of the RNA polymerase I transcription factor TIF-IA as an essential initiation factor for Pol I, wherein the DNA sequence
(a) differs from the DNA sequence from claim 2 as regards the codon sequence because of the degeneration of the genetic code;
(b) is a fragment, an allelic variant or another variant of the DNA sequence from claim 2 or 3(a).

4. A ribozyme which is complementary to the DNA sequence according to any one of claims 1 to 3 and can bind specifically to, and cleave, the RNA transcribed by this DNA sequence so as to reduce or inhibit the synthesis of the protein encoded by this DNA sequence.

5. An antisense RNA which is complementary to the DNA sequence according to any one of claims 1 to 3 and can bind specifically to the RNA transcribed by this DNA sequence so as to reduce or inhibit the synthesis of the protein encoded by this DNA sequence.

6. An expression vector which contains the DNA sequence according to any one of claims 1 to 3 or codes for the ribozyme according to claim 4 or the antisense RNA according to claim 5.

7. A host cell, containing the expression vector according to claim 6.

8. An isolated RNA polymerase I transcription factor TIF-IA or isolated protein having its biological activity as an essential initiation factor for Pol I, which is encoded by the DNA sequence according to any one of claims 1 to 3.

9. A method of producing an RNA polymerase I transcription factor TIF-IA or a protein having its biological activity according to claim 8, which comprises culturing the host cell according to claim 7 under suitable conditions and collecting the protein from the cell or the culture medium.

10. An antibody or a fragment of the antibody which binds to the RNA polymerase I transcription factor TIF-IA or a protein having its biological activity according to claim 8.

11. A medicament containing the DNA sequence according to any one of claims 1 to 3, the expression vector according to claim 6, the RNA polymerase I transcription factor TIF-IA or a protein having its biological activity according to claim 8.

12. The medicament containing the ribozyme according to claim 4, the antisense RNA according to claim 5 or the antibody or the fragment thereof according to claim 10.

13. A diagnostic method of detecting a possibly impaired TIF-IA expression, wherein a sample is contacted with the DNA sequence according to any one of claims 1 to 3 or the antibody or the fragment thereof according to claim 10 and it is then determined directly or indirectly whether the concentration, length and/or sequence of the RNA polymerase I transcription factor TIF-IA or the mRNA encoding the same differ from a control sample.

14. A diagnostic kit for carrying out the method according to claim 13, which contains the DNA sequence according to any one of claims 1 to 3 and/or the antibody or the fragment thereof according to claim 10.

## Revendications

1. Séquence d'ADN codant pour un facteur de transcription TIF-IA de l'ARN polymerase I avec la séquence d'acides aminés illustrée dans la figure 2.

2. Séquence d'ADN selon la revendication 1, qui comprend la séquence d'acides nucléiques illustrée dans la figure 2.

3. Séquence d'ADN codant pour une protéine ayant la fonction biologique du facteur de transcription TIF-IA de l'ARN polymerase I comme facteur d'initiation essentiel pour Pol I, dans laquelle la séquence d'ADN :
(a) se distingue de la séquence d'ADN de la revendication 2 dans la séquence de codons du fait de la dégénérescence du code génétique ; ou
(b) est un fragment de la séquence d'ADN de la revendication 2 ou 3 (a).

4. Ribozyme, complémentaire de la séquence d'ADN selon une des revendications 1 à 3 et pouvant se lier spécifiquement à l'ARN transcrit par cette séquence d'ADN et se séparer de celui-ci, ce qui permet de diminuer ou d'arrêter la synthèse de la protéine codée par cette séquence d'ADN.

5. ARN antisens, complémentaire de la séquence d'ADN selon une des revendications 1 à 3 et pouvant se lier spécifiquement à l'ARN transcrit par cette séquence d'ADN, ce qui permet de diminuer ou d'arrêter la synthèse de la protéine codée par cette séquence d'ADN.

6. Vecteur d'expression, contenant la séquence d'ADN selon une des revendications 1 à 3 ou codant pour le ribozyme selon la revendication 4 ou l'ARN antisens selon la revendication 5.

7. Cellule hôte, contenant le vecteur d'expression selon la revendication 6.

8. Facteur de transcription TIF-IA de l'ARN polymerase I isolé ou protéine isolée ayant son activité biologique comme facteur d'initiation essentiel pour Pol I, qui est codé(e) par la séquence d'ADN selon une des revendications 1 à 3.

9. Procédé de fabrication d'un facteur de transcription TIF-IA de l'ARN polymerase I ou d'une protéine ayant son activité biologique selon la revendication 8, qui comprend la culture de la cellule hôte selon la revendication 7 dans des conditions appropriées et l'obtention de la protéine à partir de la cellule ou du milieu de culture.

10. Anticorps ou fragment de l'anticoprs qui se lie au facteur de transcription TIF-IA de l'ARN polymerase I ou à une protéine ayant son activité biologique selon la revendication 8.

11. Médicament, contenant la séquence d'ADN selon une des revendications 1 à 3, le vecteur d'expression selon la revendication 6, le facteur de transcription TIF-IA de l'ARN polymerase I ou une protéine ayant son activité biologique selon la revendication 8.

12. Médicament, contenant le ribozyme selon la revendication 4, l'ARN antisens selon la revendication 5, ou l'anticorps ou le fragment de celui-ci selon la revendication 10.

13. Procédé de diagnostic permettant de justifier une expression TIF-IA éventuellement perturbée, selon lequel on amène un échantillon au contact de la séquence d'ADN selon une des revendications 1 à 3 ou de l'anticorps ou du fragment de celui-ci selon la revendication 10, puis on détermine directement ou indirectement si la concentration, la longueur et/ou la séquence du facteur de transcription TIF-IA de l'ARN polymerase I ou de l'ARNm codant celui-ci sont différentes par rapport à un échantillon de contrôle.

14. Kit de diagnostic permettant de réaliser le procédé selon la revendication 13, qui contient la séquence d'ADN selon une des revendications 1 à 3 et/ou l'anticorps ou le fragment de celui-ci selon la revendication 10.
